# EUROPEAN PATENT SPECIFICATION

(11) **EP 1 878 738 B1**
(45) Date of publication and mention of the grant of the patent: **27.07.2011**
(21) Application number: 06732082.0
(22) Date of filing: 19.04.2006
(51) Int. Cl.: C07H 3/06, A61K 31/702, A23L 1/30, A61P 37/08, A61P 17/00

(54) **1-KESTOSE FOR TREATING ALLERGY AND ATOPIC DERMATITIS**
1-KESTOSE ZUR BEHANDLUNG VON ALLERGIE UND ATOPISCHER DERMATITIS
1-KESTOSE POUR LE TRAITEMENT DE L'ALLERGIE ET DE LA DERMATITE ATOPIQUE

(30) Priority: 21.04.2005 JP 2005123723; 22.12.2005 JP 2005371005
(43) Date of publication of application: 16.01.2008
(73) Proprietor: THE HOKUREN FEDERATION OF AGRICULTURAL COOPERATIVES, Hokkaido, 0608651 (JP); B Food Science Co., Ltd., Aichi, 478-0046 (JP)
(72) Inventor: KOGA, Yasuhiro, Isehara-shi, Kanagawa 2591193 (JP); SHIBATA, Rumiko, Fukuoka-shi, Fukuoka 8111394 (JP); AIBA, Yuji, Muromachi 1-chome Chuo-ku Tokyo 1038330 (JP); FUKUMORI, Yasunori, Chuo-ku Sapporo-shi Hokkaido 0608651 (JP); TAKEDA, Hiroyuki, Chuo-ku Sapporo-shi Hokkaido 0608651 (JP)
(74) Representative: Oser, Andreas
(86) International application number: PCT/JP2006/308175
(87) International publication number: WO 2006/115136

(56) References cited:
- EP-A- 0 474 046
- EP-A1- 1 421 945
- WO-A2-97/02829
- JP-A- 02 235 823
- JP-A- 04 235 192
- JP-A- 09 000 199
- JP-A- 09 075 035
- JP-A- 10 000 057
- JP-A- 2000 232 878
- JP-A- 2003 201 239
- JP-A- 2003 201 239
- JP-A- 2005 306 781
- JP-A- 2005 350 444
- US-A- 5 843 922
- HOSONO AKIRA ET AL: "Dietary fructooligosaccharides induce immunoregulation of intestinal IgA secretion by murine Peyer's patch cells." BIOSCIENCE, BIOTECHNOLOGY, AND BIOCHEMISTRY APR 2003, vol. 67, no. 4, April 2003 (2003-04), pages 758-764, XP002490253 ISSN: 0916-8451
- NAKAMURA Y ET AL: "Dietary fructooligosaccharides up-regulate immunoglobulin A response and polymeric immunoglobulin receptor expression in intestines of infant mice." CLINICAL AND EXPERIMENTAL IMMUNOLOGY JUL 2004, vol. 137, no. 1, July 2004 (2004-07), pages 52-58, XP002490254 ISSN: 0009-9104
- ISOLAURI E ET AL: "PROBIOTICS IN THE MANAGEMENT OF ATOPIC ECZEMA" CLINICAL AND EXPERIMENTAL ALLERGY, BLACKWELL SCIENTIFIC PUBLICATIONS, LONDON, GB, vol. 30, 1 November 2000 (2000-11-01), pages 1604-1610, XP001064231 ISSN: 0954-7894
- ITO M. ET AL.: 'Igaku Shoin Igaku Daijiten' IGA & IGA KESSONSHO NEN 3 GATSU 1 NICHI 2003, page 2, XP003004654

## Description

### BACKGROUND OF THE INVENTION

### 1. Field of the Invention

The present invention generally relates to an composition effective for the inihibition of allergic diseases, and particularly to an allergy-suppressive composition, an allergy-suppressive food and an allergy-suppressive agent comprise 1-kestose as the active ingredient according to the claims suitable for the prevention of the development and treatment of allergic diseases since 1-kestose has an effect of increasing the production of an immunoglobulin A (IgA), inhibiting the production of an immunoglobulin E (IgE) and activating the growth of an intestinal *Bifidobacterium.*

### 2. Description of the Related Art

Allergic diseases are caused when the immune system shows a malfunction in self-protection against enemies such as foreign substances. Allergic diseases, such as food allergy, pollinosis and atopic dermatitis, are found in patients of various ages and their regions and symptoms are different.

Allergic diseases are normally found in the form of allergy march in which main symptoms change by age. Specifically, patients with atopic disposition develop atopic dermatitis in infancy, suffer subsequently from bronchial asthma in early childhood and allergic rhinitis as they reach adulthood. These successive allergy march symptoms have become a big social problem, requiring prompt and effective measures. In fact, proper treatment of atopic dermatitis in early-stage such as infancy can prevent further development of allergic diseases.

Allergic diseases, which are attributed mainly to an IgE antibody, can be prevented by inhibiting the production thereof.

Also, it is known that the IgA antibody is found in human intestinal tracts and particularly a secretory IgA stimulates the immune system. The immune system finds foods to be taken essentially foreign and it is mostly tolerant of foreign proteins absorbed into the intestinal tract. This intestinal immune tolerance can be mainly achieved by the IgA antibody. Thus, the activation of said IgA might be an additional allergy preventive measure.

Moreover, the development of allergic diseases is associated with the composition of enterobacterial flora before the problem develops and it is reported that in young children with allergic symptoms, the bifidobacterial count of enterobacterial flora in newborn infancy is lower than in healthy young children. In view of this observation, it seems an efficient means to improve the composition of enterobacterial flora to prevent allergic diseases.

Meanwhile, the use of anti-allergic drugs for directly treating the affected region or preventing the binding of foreign matter with IgE requires doctor's prescription. Also, it is difficult to determine to continue or discontinue its taking and many cases with side-effects have been reported.

It is also indicated that some foods have an effect of inhibiting allergic symptoms, but there are various drawbacks such as undefined effects of components extracted from a plant, required careful bacteriologic control and many types of foods with poor taste quality.

Most atopic dermatitis patients, who are mainly infants and young children sensitive to food safety, must take completely safe foods and highly-processed foods to intake easily.

Conventional inventions concerning substances having allergy-suppressive functions are proposed, like an allergy-preventing material containing fructooligosaccharide as disclosed in a Japanese Unexamined Patent Publications No. 08-157379 and an immunostimulating food containing fructooligosaccharide as disclosed in another Japanese Unexamined Patent Publication No. 2003-201239.
A. Hosono et al. report in Biosci. Biotechnol. Biochem. 67 (4), 758-764 (2003) about the use of a fructooligosaccharide (FOS)-mixture of 42% 1-kestose, 46% nystose and 9% 1F-β-fructofuranosylnystose to increase to production of IgA.

### SUMMARY OF THE INVENTION

However, the above-mentioned invention in the Japanese Unexamined Patent Publication No. 08-157379 focuses on a method by promoting magnesium absorption, with no descriptions of an effect on the immune system. Another conventional invention in the No. 2003-201239 shows a description of an immunostimulating effect by a mixture of various oligosaccharides, but it doesn't mean the identification of a factor in the composite, particularly no description of an effect of kestose. According to experiments conducted by the inventors using oligosaccharide with a low purity of 1-kestose, the inhibition of the production of an IgE antibody was not achieved, and thus they shifted their focus onto an effect of inhibiting the allergy by 1-kestose.

Moreover, the inventors found an effect of improving the composition of enterobacterial flora by proliferating *Bifidobacterium* (known as useful intestinal bacterium) by oligosaccharide with 1-kestose as a main ingredient and focused on the resulting allergy-suppressive effect for atopic dermatitis, etc.

Kestose is an oligosaccharide composed of three monosaccarides, a substance contained in vegetables and grain with no side effects by abundant dietary experience. Crystallized 1-kestose is low in hygroscopicity and its quality of taste and solubility are equivalent to sugar. Therefore, this material is suitable for infants and young children to take on a daily basis.

To solve the aforementioned problems, the present invention therefore provides a composition for use according to claim 1. Preferred embodiments of the invention are provided by claims 2 to 8. The present invention specifically provides an allergy-suppressive composition, an allergy-suppressive food and an allergy-suppressive agent using an oligosaccharide with 1-kestose as main ingredient, wherein the amount of 1-kestose is larger than that of nystose, by confirming that 1-kestose has an effect of increasing the production of an IgA antibody, an effect of inhibiting the production of an IgE antibody, an effect of activating the growth of an intestinal *Bifidobacterium* and an effect of ameliorating atopic dermatitis in infants and young children in tests in human.

The allergy-suppressive composition according to the present invention comprises an oligosaccharide with 1-kestose as main ingredient wherein the amount of 1-kestose is larger than that of nystose.

The allergy-suppressive composition according to the present invention comprises 1-kestose as an active ingredient and has an effect of increasing the production of an IgA antibody.

The allergy-suppressive composition according to the present invention comprises 1-kestose as an active ingredient and has an effect of inhibiting the production of an IgE antibody.

The allergy-suppressive composition according to the present invention comprises 1-kestose as an active ingredient and has an effect of activating the growth of an intestinal *Bifidobacterium* in infants and young children.

The allergy-suppressive composition according to the present invention comprises 1-kestose as an active ingredient and has an effect of ameliorating atopic dermatitis in infants and young children.

In this invention, the amount of 1-kestose contained is larger than that of nystose.

Preferably in this invention, 1-kestose is a crystallized kestose with a purity of 95 % or more.

Also, in this invention, it is desirable that a daily effective amount (dose) be 0.015 (g/kg-body weight) or more.

Moreover, the allergy-suppressive food according to the present invention comprises the above-mentioned allergy-suppressive composition.

The allergy-suppressive agent according to the present invention comprises the above-mentioned allergy-suppressive composition.

According to the present invention, 1-kestose can provide an effect of inhibiting the production of an allergy etiology, IgE (IgG1) antibody, an effect of increasing the production of an IgA antibody leading to intestinal immune tolerance and an effect of preventing and treating allergic diseases. Also, it can improve the composition of enterobacterial flora, prevent and treat atopic dermatitis in infants and young children and prevent of the development of subsequent allergic diseases.

### BRIEF DESCRIPTION OF THE DRAWINGS

The above and other objects of the invention will be seen by reference to the description taken in connection with the accompanying drawings, in which:
Figure 1 is a table showing the results of IgA concentration in feces measured before and after taking 1-kestose in Example 1;
Figure 2 is a graph of the results of Figure 1 in Example 1;
Figure 3 is a table showing the intestinal bacterial count before taking 1-kestose in Example 1;
Figure 4 is a table showing the intestinal bacterial count after taking 1-kestose in Example 1;
Figure 5 is a graph showing the change in *Bifidobacterium* count in intestinal tract before and after taking 1-kestose in Example 1;
Figure 6 is a graph showing the change in occupancy of intestinal *Bifidobacteria* by taking 1-kestose in Example 1;
Figure 7 is a graph showing an effect of inducing oral immune tolerance based on the change in IgG1 amount by orally administering 1-kestose in Example 2;
Figure 8 is a table showing an effect of inducing oral immune tolerance based on IgE amount by orally administering 1-kestose in Example 2;
Figure 9 is a table showing the results of IgE amount in human serum before and after taking 1-kestose in Example 3;
Figure 10 is a table showing the composition of saccharide used in an experiment in Example 4;
Figure 11 is a graph showing the comparison of the effect of improving enterobacterial flora by each substance comprising fructooligosaccharide in Example 4;
Figure 12 is a table showing the comparison of the effect of inhibiting IgE by 1-kestose and fructooligosaccharide in Example 5;
Figure 13 is a table showing the experimental results to determine the effective amount of 1-kestose in Example 6;
Figure 14 is a graph showing the bifidobacterial count relative to the effective amount of 1-kestose in Example 6; and
Figure 15 is a table showing clinical findings, the occupancy of *Bifidobacteria* and side effect before and after taking 1-kestose in Example 7.

### DETAILED DESCRIPTION OF THE PREFERRED EMBODIMENT

The inventors of this invention found a role of oligosaccharide that contains 1-kestose with a high purity in proliferating *Bifidobacterium,* even in an intestinal environment in which harmful bacteria such as bacteria of *Clostridium* are more predominant than *Bifidobacterium* in a Japanese Unexamined Patent Publication No. 2004-126086. In this invention, however, their further researches on the availability of 1-kestose led to an allergy-suppressive effect in a composition that contain 1-kestose with a high purity.

A 1-kestose used in this embodiment is a trisaccharide, oligosaccharide composed of monomolecular glucose and bimolecular fructose. An oligosaccharide with 1-kestose as a main ingredient can be produced by reacting an ingredient sucrose with various enzymes to obtain a 1-kestose-containing composition as disclosed in Japanese Unexamined Patent Publication No. 58-201980, increasing the purity of 1-kestose by chromatographic separation as disclosed in Japanese Unexamined Patent Publication No. 2000-232878 and crystallizing 1-kestose with a purity of 95% or more as disclosed in Japanese Examined Patent Publication No. 06-070075. A crystallized oligosaccharide, having 1-kestose as a main ingredient produced in the conventional methods, is an indigestible substance of excellent solubility and low energy with effects of increasing the production of IgA and inhibiting the production of IgE. The composition with 1-kestose of this embodiment as an active ingredient is most preferably 1-kestose described in New Food Material Efficient Use Technology Series No. 13 "1-kestose" (issued by Japan Confectionery and Innovative Food Ingredients Research Center). The results of each Example will be described as follows to demonstrate allergy-suppressive effects of said 1-kestose.

### [Example 1]

### "Example 1: Experiment for confirming the effect on IgA production"

In Example 1, the effect of 1-kestose on IgA production was experimentally investigated.

The composition, containing 1-kestose used in this Example 1, is of a 1-kestose purity of 98%, composed of 1-kestose (98 weight %), nystose (1.0 weight %) and sucrose (1.0 weight %). An allergy-suppressive composition, having 1-kestose as an active ingredient, was given to 4 adult males having allergic symptoms, with each dose of 3g for one month. The feces before and after the composition was taken were collected to measure IgA and intestinal bacteria in the feces.

To measure IgA, the collected feces were diluted 20 times with phosphate buffered saline (PBS) and supernatant was collected by a centrifugal separator. Then, the liquid was diluted 10 times with water to make a sample for measurement. Also, an anti-human IgA mouse monoclonal antibody (product of Nacalai Tesque Inc.), and a horseradish peroxidase (HRP)-labeled antibody by labeling the antihuman IgA mouse monoclonal antibody with HRP were prepared. Afterward, IgA was measured using ELISA method.

Meanwhile, to measure intestinal bacteria, a sample liquid for measurement was dripped on a flat-plate agar medium by 0.05ml and applied thereon. After anaerobic culture of the sample at 35°C for 3 to 5 days, the bacteria were identified and bacterial count was found.

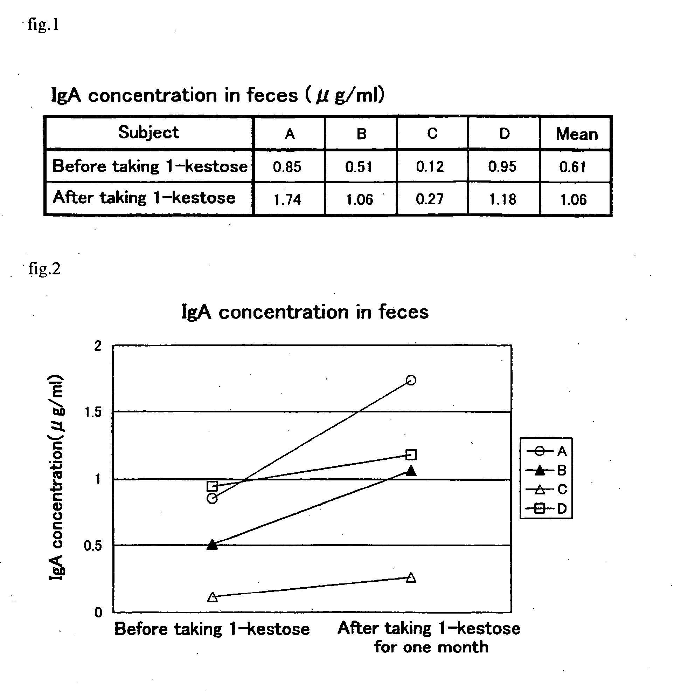

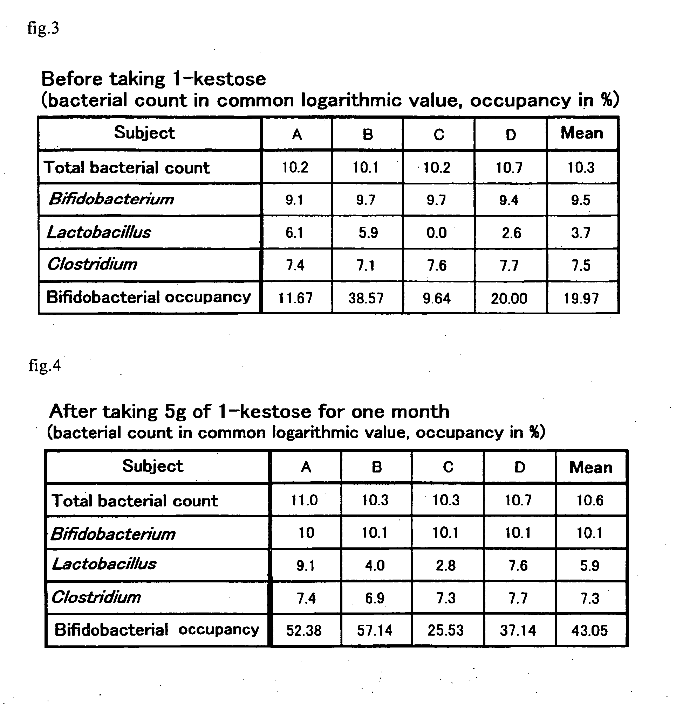

Figures 1 and 2 show the results of IgA measured in Example 1 and Figures 3 to 6 show the results of intestinal bacteria measured. As shown in Figures 1 and 2, the IgA concentration in feces increased in each subject by taking the 1-kestose-containing composition for one month, and particularly twice or more in subjects A to C. The mean IgA concentration in each subject changed from 0.61 to 1.06 (µg/ml), showing an increase of 1.7 times or more. As a result, the effect of taking 1-kestose was found to increase intestinal IgA.

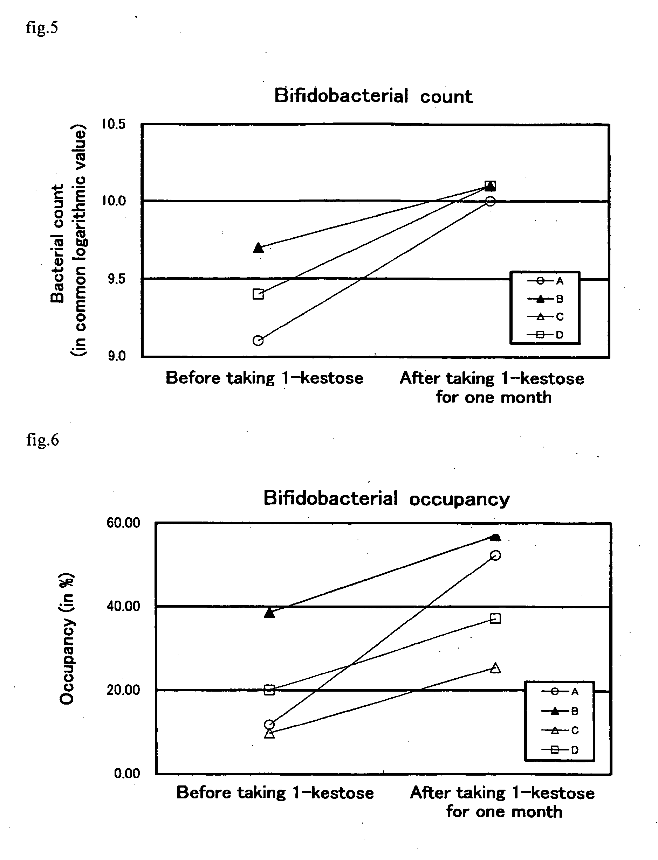

Next, the results of intestinal bacteria measured will be discussed. As for the intestinal bacteria, *Bifidobacterium* and *Lactobacillus* were identified as a useful intestinal bacterium and *Clostridium* as a harmful bacterium. Figure 3 is a table showing each bacterial count and bifidobacterial occupancy before taking 1-kestose and Figure 4 is a table showing each bacterial count and bifidobacterial occupancy after taking 1-kestose for one month. Figures 5 and 6 are graphs of *Bifidobacterium* data. As shown in each graph, the bifidobacterial counts in subjects A to D were 10^{9.1}, 10^{9.7}, 10^{9.7}, 10^{9.4}, respectively, but they all increased to 10^{10.0}, 10^{10.1}, 10^{10.1} and 10^{10.1}, by taking 1-kestose for one month. The mean bifidobacterial count remarkably increased from 10^{9.5} to 10^{10.1}.

In subjects B and C, the *Clostridium* count declined from 10^{7.1} to 10^{6.9}, and 10^{7.6} to 10^{7.3}, respectively. The mean *Clostridium* count decreased from 10^{7.5} to 10^{7.3}. The bacterial count was obtained with 1g of wet weight-feces.

The bifidobacterial occupancy relative to the total bacterial count showed a significant increase in each subject and the mean occupancy increased from 19.97% to 43.05 % by taking 1-kestose for one month, resulting in the dominance of *Bifidobacterium* in the intestinal tract. The subjects A and B, in particular, showed an increase in bifidobacterial count from 11.67% to 52.38% and 38.57% to 57.14%, respectively, exceeding one half of each occupancy and with remarkable improvement in intestinal environment. Since the intestinal tract is normally occupied by *Bacteroides* (approx. 40%) and other types of bacteria (approx. 20%), *Bifidobacterium* can be the most dominant bacterium with an occupancy of over 40%. This obvious effect by *Bifidobacterium* demonstrates the improvement in intestinal environment by taking 1-kestose according to the results of this Example 1.

The discussion of the relationship between *Bifidobacterium* and allergy showed the effect of activating immune cells such as macrophage and NK cell by continuing to orally take *Bifidobacterium.* According to conventional studies, Gram-positive bacteria like *Bifidobacterium* induce Th1 cells that inhibit allergic diseases in the intestinal immune system. Consequently, the activation of the growth of an intestinal *Bifidobacterium* can have an effect of preventing and treating the allergy.

From the results of the above Example 1, 1-kestose has an effect of increasing the production of IgA, bifidobacterial count and intestinal occupancy is a suitable substance to improve the intestinal environment.

### [Example 2]

### "Example 2: Experiment for confirming the effect on the production of IgG1 and IgE"

In Example 2, to examine the effect of 1-kestose on the production of IgG1 and IgE, 1-kestose was administered to germfree mice to measure IgG1 and IgE amounts in the serum before and after giving 1-kestose

In this Example 2, the mice used in the experiment were male rats with 4 weeks of age born and grown in a germfree condition (BALB/c), with 10 to 15 rats as a group. These mice were orally provided with 0.5ml of a liquid obtained by diluting feces of human infants 100 times and observed in a germfree condition for two weeks. Afterward, 5mg of ovalbumin (OVA) was orally administered to the mice for 4 days. To examine the existence of immune tolerance, a liquid obtained by adding 0.1 mg of aluminum hydroxide gel to 1µg of OVA was injected into the abdominal cavity, 1, 3, 5 and 7 weeks after orally administering OVA. These human-flora associated mice were separated into 1-kestose-administered group and non-administered control group and anti-OVA IgG1 and anti-OVA IgE in the serum were measured by ELISA method, 9 weeks after administering 1-kestose.

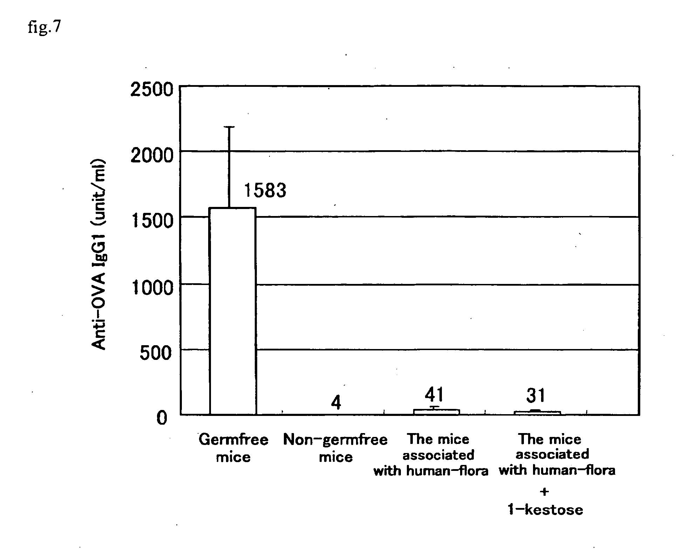

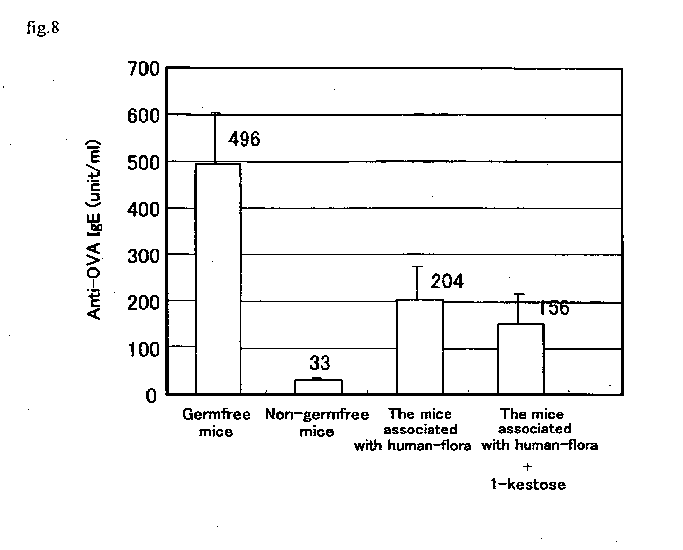

As a result, as shown in Figure 7 and Figure 8, the germfree mice showed the production of antibodies, with an IgG1 (anti-OVA IgG1) of 1583 (unit/ml) and an IgE (anti-OVA IgE) of 496 (unit/ml). On the other hand, the non-germfree mice showed 4 (unit/ml) of anti-OVA IgG1 and 33 (unit/ml) of anti-OVA IgE, significantly lower values than said germfree mice, resulting in a complete introduction of oral immune tolerance. It was thus confirmed that test system for determining the effect of oral immune tolerance (OVA and aluminum hydroxide gel) is appropriate.

In the human-flora associated mice by fixing human faecalis to the germfree mice, anti-OVAIgG1 was 41 (unit/ml) and anti-OVA IgE was 204 (unit/ml) before taking 1-kestose, each having a lower value than that of the germfree mice. In the group in which 1-kestose was administered to the human-flora associated mice, anti-OVA IgG1 decreased to 31 (unit/ml) and anti-OVA IgE to 156 (unit/ml), resulting in much lower values than those of the germfree mice. This observation demonstrated the effect of 1-kestose on ensured introduction of oral immune tolerance.

From the results of the above Example 2, it was confirmed that 1-kestose has an effect of inhibiting the production of IgG 1 and IgE and is a suitable substance to ensure oral immune tolerance.

### [Example 3]

### "Example 3: Experiment for measuring IgE amount in the human serum before and after administering 1-kestose"

In this Example 3, to examine the effect of 1-kestose on IgE amount in the human serum, the subjects were given 1-kestose to measure nonspecific IgE amount in the serum before and after taking 1-kestose.

The subjects were 2 volunteer patients with allergic symptoms (adult male and female each). They took 3g of 1-kestose for one month and blood components like nonspecific IgE in the serum were examined.

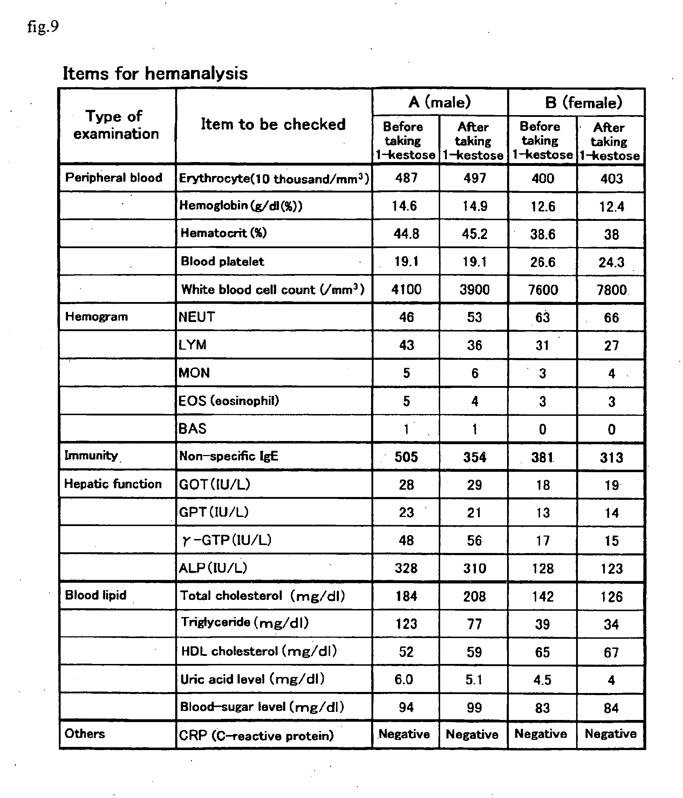

As a result, as shown in Figure 9, the nonspecific IgE of the adult male decreased from 505 to 354 (IU/ml) by taking 1-kestose for one month, showing an improvement under a standard value of 366. Also, the nonspecific IgE of the adult female changed from 381 to 313 (IU/ml), with a similar improvement under the standard value. C-reactive protein (CRP) value was negative, both before and after taking 1-kestose, with no effect on hepatic functions, etc., other organs and blood components.

From the results of the above Example 3, it was confirmed that 1-kestose has an effect of inhibiting IgE production in human as well.

### [Example 4]

### "Example 4: Experiment for comparing the effect of improving enterobacterial flora in fructooligosaccharide composition"

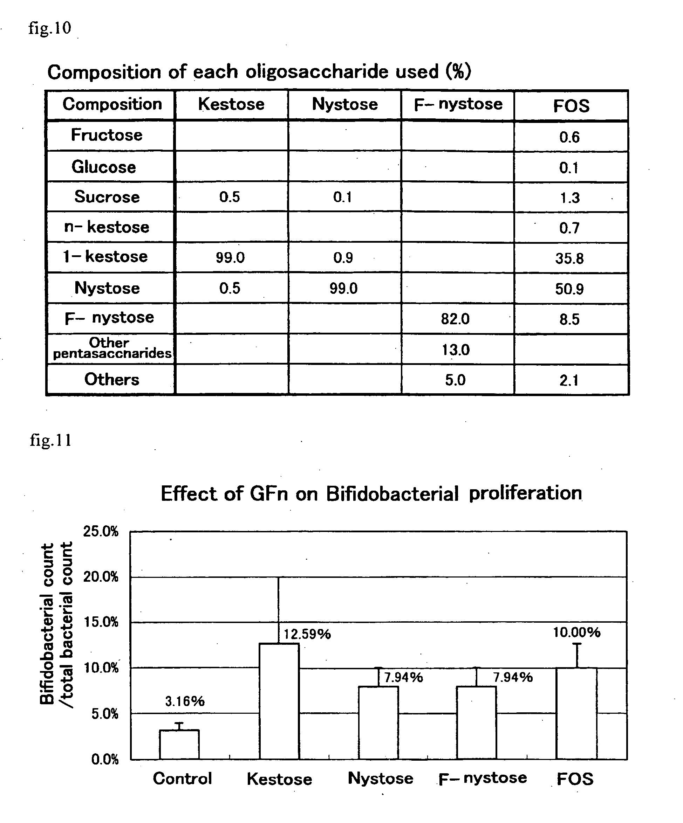

In this Example 4, the effect of improving the enterobacterial flora of each composition composed of fructooligosaccharide was compared, using germfree mice. The fructooligosaccharide is composed of kestose (trisaccharide), nystose (tetrasaccharide) and F-nystose (pentasaccharide). The kestose used in this Example 4 is composed of 1-kestose (99.0%), nystose (0.5%) and sucrose (0.5%). The nystose is composed of nystose (99.0%), 1-kestose (0.9%) and sucrose (0.1 %). The F-nystose is composed of F-nystose (82.0%), other pentasaccharides (13.0%) and other components (5.0%). Additionally, control groups of mice dosed with sterile water and dosed with usual fructooligosaccharide (1-kestose: 35.8 weight %, nystose: 50.9 weight %, F-nystose: 8.5 weight %) were prepared and their data were obtained for comparison. Figure 10 shows the composition of each oligosaccharide used in the experiment. Each oligosaccharide was orally administered to germfree mice for 5 days to determine the proportion of *Bifidobacterium* proliferated to total bacterial count.

As a result, as shown in Figure 11, the proportion in kestose was the highest with 12.59%, followed by 7.94% in both nystose and F-nystose. The proportion in fructooligosaccharide was 10.00%. Compared with the mice of the control groups, there found an improvement in bifidobacterial occupancy and thus intestinal environment, thereby confirming the most significant effect of improving the intestinal environment by kestose.

From the results of the above Example 4, it was confirmed that 1-kestose has the most significant effect of proliferating *Bifidobacterium* as a composition of fructooligosaccharide and is a suitable substance to improve the intestinal environment.

### [Example 5]

### "Example 5: Comparative experiment on the positive effects of 1-kestose and fructooligosaccharide "

The analysis of the results of the above Example 4 showed the most significant effect of 1-kestose on *Bifidobacterium* proliferation as a composition of fructooligosaccharide and it was more favorable than that of fructooligosaccharide. From this observation, making the purity of 1-kestose higher seems effective for improving intestinal environment. To confirm a direct effect of inhibiting the allergy, the effect of inhibiting IgE by taking 1-kestose and fructooligosaccharide was experimentally discussed in Example 5.

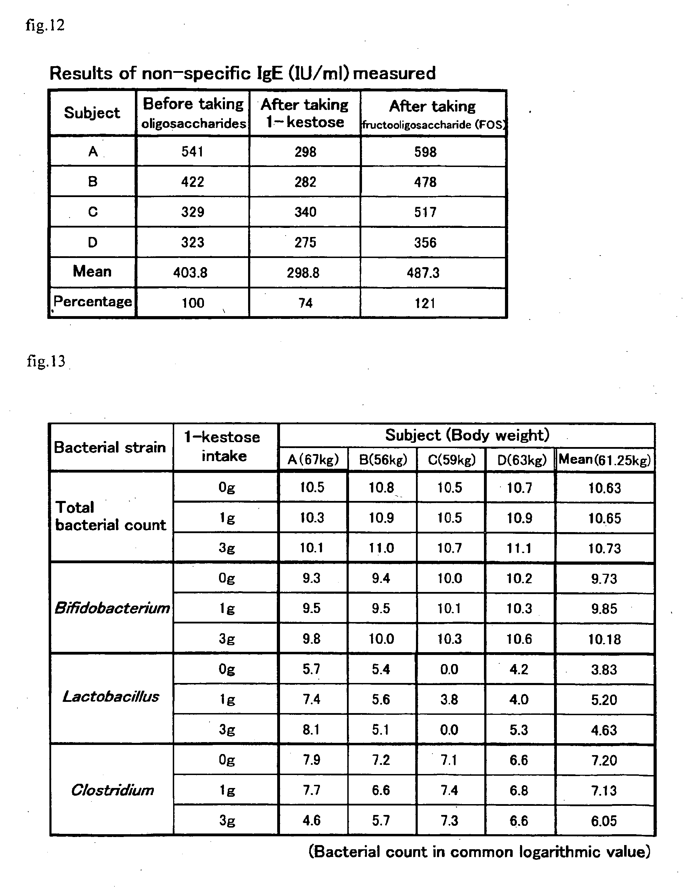

In this Example 5, the subjects were 4 adult females (from 27 to 45 years old) with daily allergic symptoms. They took 1-kestose and fructooligosaccharide and nonspecific IgE of each subject was measured. In this experiment, nonspecific IgE before taking the substances was measured and then the subjects took 3g of 1-kestose after eating once a day for 4 weeks. The blood was collected 5 weeks later to measure nonspecific IgE and afterward they took no substances for 2 weeks. Then, the subjects took 3g of fructooligosaccharide after eating once a day for 4 weeks and the blood was collected. The 1-kestose used in this Example 5 has a purity of 95% or more, and the commercially-available fructooligosaccharide is a nystose-dominant oligosaccharide composed of nystose (51 %) and 1-kestose (36%). In the experiment, the subjects took less health foods like prebiotics and probiotics containing other oligosaccharides and *Lactobacillus.* Figure 12 shows the results of Example 5.

As shown in Figure 12, three of the 4 subjects showed a significant decrease in nonspecific IgE by taking 1-kestose, but fructooligosaccharide intake increased nonspecific IgE in all the subjects.

Specifically, the nonspecific IgE in the subject A changed from 541 to 298 (IU/ml) by taking 1-kestose, resulting in a decrease by approximately 45 % Likewise, the subject B changed the nonspecific IgE from 422 to 282 (IU/ml), with a decrease of 32% or more, and the subject C changed the nonspecific IgE from 323 to 275 (IU/ml), with a decrease of approximately 25%.

Meanwhile, fructooligosaccharide intake increased the nonspecific IgE in the subject A to 598 (IU/ml) by 10% or more. The subject B also increased the nonspecific IgE to 478 (IU/ml), with an increase of 13% or more, and the subject D marked an increase of 11% or more to 356 (IU/ml).

The subject C showed no decrease in nonspecific IgE, but an increase of about 3 % by taking 1-kestose. The fructooligosaccharide intake increased this value by 57% or more. Thus, it was confirmed that 1-kestose has a more dominant role in increasing nonspecific IgE than fructooligosaccharide.

According to the above results of this experiment, the mean nonspecific IgE changed from 403.8 to 298.8 (IU/ml) by taking 1-kestose, resulting in a decrease of approximately 26% or more. However, fructooligosaccharide intake led to no such decrease, with an increase of 20% or more to 487.3 (IU/ml).

From the results of the above Example 5, 1-kestose has an effect of inhibiting nonspecific IgE, but fructooligosaccharide intake can provide no such effect and on the contrary, it increases the production of nonspecific IgE. In view of the results of Example 4 as well, it seems that nystose and F-nystose composed of fructooligosaccharide cancel the effect of inhibiting nonspecific IgE by 1-kestose and increasing IgE production.

From these observations, it was confirmed that the effect of inhibiting nonspecific IgE or allergic diseases can be provided by a composition containing 1-kestose with a high purity in fructooligosaccharide, not by a nystose-based composition. The 1-kestose contained in fructooligosaccharide is required to have a higher purity larger than nystose as a first composition, and the purity is preferably 90% or more, and more preferably 95% or more. More specifically, it is necessary that 1-kestose can provide the effect of increasing the production of IgA antibody or the effect of inhibiting the production of IgE antibody in high purity.

### [Example 6]

### "Example 6: Experiment for determining the effective amount of 1-kestose"

According to the results of each above-mentioned Example, it was confirmed that an adult can have the effect of 1-kestose as prebiotics with a daily dose of 3g. In addition, volunteer subjects showed the effect of improving bowel movement with a daily dose of 2g in an experiment. In this Example 6, some volunteer subjects took part in an experiment to demonstrate if they show the effect of improving the intestinal environment with a smaller daily dose of 1g. The subjects were 4 health adult males whose age ranges from 38 to 56. The subject A is 67kg, subject B is 56kg, subject C is 59kg and subject D is 63kg in weight.

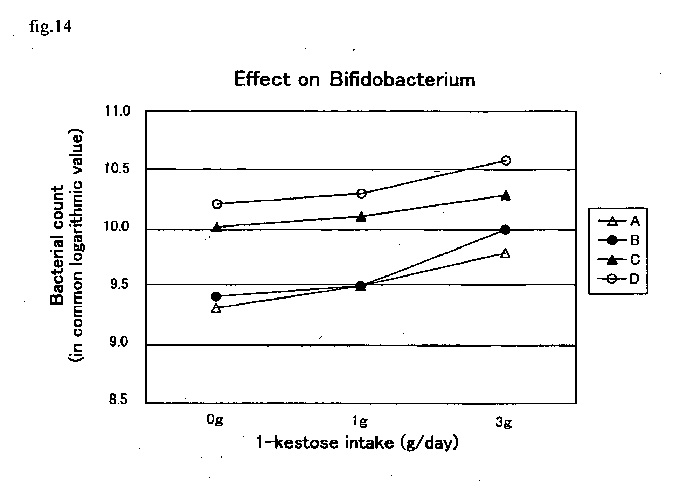

In the experiment, feces were first collected from each subject before taking 1-kestose, and counts of *Bifidobacterium, Lactobacillus, Clostridium* and total bacteria were measured. Then, they took 1g of 1-kestose once a day for 2 weeks. 15 days later, feces were collected from each subject to measure intestinal bacteria. Afterward, they took no 1-kestose for one week. From the 22^{nd} day, they took 3g of 1-kestose once a day for two weeks, and feces were collected from each subject on the 36^{th} day to measure intestinal bacteria. Figure 13 and Figure 14 show the results.

As shown in Figure 13 and Figure 14 in logarithmic value, all the subjects showed an increase in *Bifidobacterium* by taking 1g or 3g of 1-kestose. The mean bifidobacterial count increased from 10^{9.73} to 10^{9.85} by taking 1g of 1-kestose once a day, and it further increased to 10^{10.18} with a daily intake of 3g. From this result, the effective 1-kestose amount for an adult male with a weight of at least 60kg was found 1g per day. The bacterial count was obtained with 1g wet weight of feces.

Meanwhile, the 1-kestose intake with no side effect was examined. After the volunteer subjects took 10g of 1-kestose once a day, they developed no diarrhea with this intake and no effect on organs and blood component was confirmed. In another experiment using other volunteers, a daily 1-kestose intake of 0.4g per 1kg body weight (or 24g a day for a patient with a weight of 60kg) showed no diarrhea symptoms in the subject.

From the results of the above Example 6, the effective amount of 1-kestose per 1kg body weight was 0.015 (g/kg body weight) in subject A, 0.018 (g/kg body weight) in subject B, 0.017 (g/kg body weight) in subject C and 0.016 (g/kg body weight) in subject D. The mean value was 0.016 (g/kg body weight). Consequently, if adult males take at least 0.015 (g/kg body weight) or more of 1-kestose a day as the effective amount, they can show an effect aimed in this invention. On the other hand, a daily intake of 0.4 (g/kg body weight) or less causes no symptoms like diarrhea.

Thus, the dose of 1-kestose with the effect of treating atopic dermatitis of this invention can be determined accordingly in view of patient's conditions like age and weight, administration route, disease symptoms and severity. For example, the daily 1-kestose dose for an adult (body weight: 60kg) is preferably 0.9 to 24g and more preferably 2 to 6g in oral administration. For a young child (body weight: 10kg), the daily 1-kestose dose is preferably 0.15 to 4g and more preferably 0.3 to 3g in oral administration. As for rectal administration, the daily 1-kestose dose for an adult (body weight: 60kg) is preferably 0.9 to 24g and more preferably 2 to 6g.

### [Example 7]

### "Example 7: Clinical test in atopic dermatitis patient using 1-kestose"

According to the results of each above-mentioned Example, it was confirmed that 1-kestose has effects of inhibiting an IgE antibody as allergy causative agent, increasing the production of IgA antibody and activating the proliferation of *Bifidobacterium* in adults with daily allergic symptoms. Then, Example 7 focuses on possibilities for treating atopic diathesis in allergy patients in infancy and preventing various subsequent allergic symptoms in a clinical test, using infants with atopic dermatitis symptoms.

The test subjects were 13 infants with atopic dermatitis and mild and severe eruption (age: 0 to 3 years old) in which food allergic symptoms were accompanied. The test was approved by parents of the subjects.

In the clinical test, the subjects were kept under observation for 2 weeks and they took 1-kestose with a purity of 98% mixed with water or juice once a day for 12 weeks. The subjects at age 1 or under were provided with 1g of 1-kestose a day and those at age 2 to 3 took 2g of 1-kestose a day. The test subjects used no external preparation of steroid for one month or more before taking 1-kestose. During the test, the subjects were treated mainly with a humectant and took food allergen removing food.

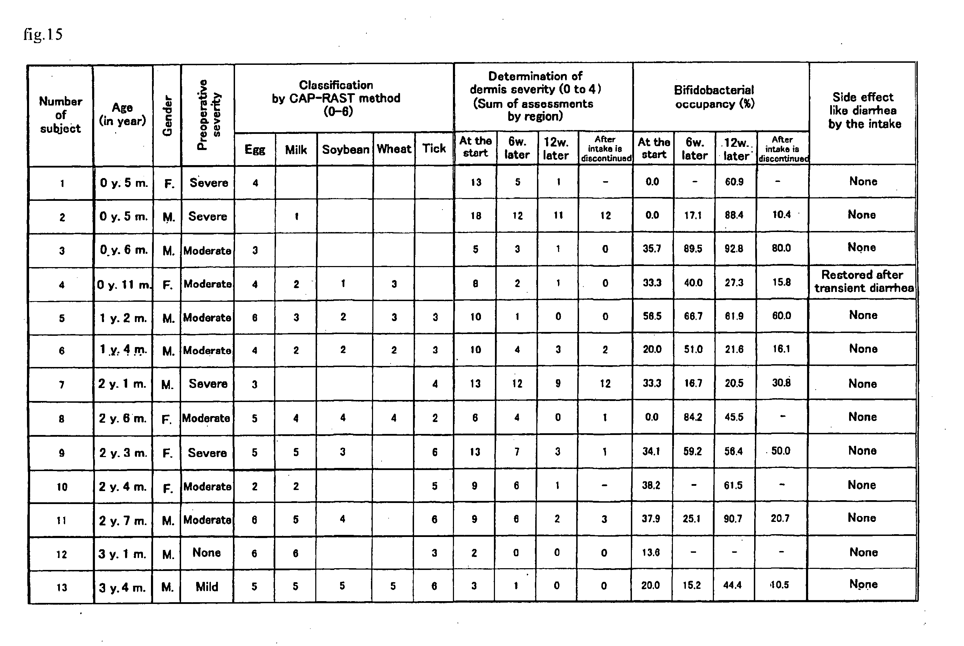

The subjects were diagnosed at the start of, 6 and 12 weeks after taking 1-kestose, and 4 to 8 weeks after the intake completion to assess the clinical test results (examination of dermis severity and stool samples). The inspection 4 to 8 weeks after the intake completion was aimed at examining drug efficacy sustaining effect. In the stool sample inspection, feces were collected form the subjects to calculate bifidobacterial occupancy in the feces. The eruption severity was examined using Severity Determination Method by Region of Severity Discussion Committee of Japanese Dermatological Association, in which by separating the entire body into 5 regions: head, anterior and posterior parts of the trunk, upper and lower extremities, the sum of comprehensive assessments is calculated in each region (on 0 to 4 scales). Figure 15 shows the results.

According to the determination of dermis severity as shown in Figure 15, all the subjects who took 1-kestose showed a significant improvement in eruption. In particular, 6 (subjects 3, 4, 5, 8, 12 and 13) of 13 subjects completely removed eruption soon, 4 to 8 weeks after taking 1-kestose for 12 weeks. Seven (subjects 3, 4, 5, 6, 9, 12 and 13) of 11 subjects showed no deterioration or recurrence 4 to 8 weeks after taking 1-kestose for 12 weeks, resulting in continued effect of improving eruption by 1-kestose. This effect is the most important in the treatment of atopic dermatitis, and it was confirmed that 1-kestose plays a significant role in improving the eruption. Although this test was carried out in an exacerbation stage of early summer, such an improving effect can be evaluated, in addition to no symptom exacerbation.

The bifidobacterial occupancy in the feces increased in 10 (subjects 1, 2, 3, 5, 6, 8, 9, 10, 11 and 13) of 12 subjects by taking 1-kestose for 12 weeks, except for subject 12 who failed to provide a stool sample, with an improvement in composition of enterobacterial flora. The mean bifidobacterial occupancy in 9 subjects who were able to participate in all 4 stool sampling inspections was 30.1 % before taking 1-kestose, but it remarkably increased to 42.3% and 56.0%, 6 and 12 weeks after taking 1-kestose, respectively. With a bifidobacterial occupancy of usual infants at 50% or more, it is found that the effect of increasing bifidobacterial occupancy by 1-kestose in this clinical test was quite noteworthy.

Nevertheless, few subjects maintained a high level of the mean bifidobacterial occupancy 4 to 8 weeks after taking 1-kestose for 12 weeks, thereby indicating an overall declining trend with a reduced mean occupancy of 32.7%. Consequently, it is desirable that 1-kestose be continuously taken to maintain bifidobacterial occupancy. As mentioned above, the effect of improving eruption was found despite the decline in bifidobacterial count.

The eruption observations and bifidobacterial occupancy were statistically analyzed. The eruption at the start of, 6 and 12 weeks after taking 1-kestose and 4 to 8 weeks after intake completion, were defined as 4 points and compared between 2 groups according to multi-group test model method. As a result of this parametric test (Fisher's PLSD test), there were significant differences between at the start of and 12 weeks after taking 1-kestose, and between at the start of and 4 to 8 weeks after intake completion. In this comparison, p values were 0.0022 and 0.0025, respectively. Next, as in the eruption analysis, bifidobacterial occupancy (proportion of *Bifidobacterium* to total bacterial count) was analyzed between the 2 groups. There was a significant difference between at the start of and 12 weeks after taking 1-kestose, with p value of 0.0308.

Meanwhile, 1-kestose intake led to no observation of side effects like diarrhea and useful results like improved bowel movement were obtained in many subjects. Subject 4 had a transient diarrhea probably because of a side effect by taking 1-kestose, but this symptom was reduced during the intake.

From the results of the above Example 7, it was confirmed that 1-kestose intake not only improved the eruption, but also increased bifidobacterial occupancy in feces. 4 to 8 weeks after taking 1-kestose for 12 weeks, the bifidobacterial occupancy in feces declined to the value at the start of the intake, but the effect of improving the eruption was maintained. Thus, it was also confirmed that 1-kestose is effective in treating and preventing dermis symptoms in infants such as dry skin, crack, erythema, abrasion, keratin peeling and atopic dermatitis having symptoms like itching sensation and pain.

### "Example 8: Example of 1-kestose-containing food production"

### [Example of food production 1: 30 1-kestose-containing biscuits, content; 1 g/piece]

The following ingredients were mixed until the moisture was removed: 100g of Sakusaku flour (sago starch), 50g of tapioca flour (cassava starch), 9g of pumpkin powder (steamed, dried and powdered pumpkin), 1g of sodium bicarbonate, 30g of 1-kestose, 12g of cottonseed shortening (used instead of salt-free butter, product of Tsuji Safe Food for allergic diseases), 24g of grape seed oil (product for allergic diseases), little touch of lemon juice and one drop of vanilla extract. Then, 125cc of boiling water was added thereto slowly and the dough was kneaded as hard as the ear lobe.

Then, the dough was put on an oven paper, kept dry by covering it in a plastic wrap and spread into 5mm-thick dough with a rolling pin. The wrap was removed, unmolded with a favorite shape, and the rest of the dough was taken off from the oven paper and placed on another oven paper to be unmolded again. The oven paper with the unmolded dough was put on a top plate and the dough was baked on the oven with a temperature of 180°C for 15 minutes to make biscuits.

Compared with biscuits that were produced using the equal amount of sugar as 1-kestose, the product was less sweet but of the same palate feeling. With the total amount of 1-kestose and sugar at 30g, biscuits were successfully produced even when the mixing ratio of 1-kestose and sugar was changed.

### [Example of food production 2: 12 1-kestose-containing rice-flour dumplings, content; 1.25g/piece]

The following ingredients were mixed: 100g of Sakusaku flour (sago starch), 100g of tapioca flour (cassava starch), 15g of 1-kestose and 15g of sugar. Then, 220cc of boiling water was added thereto slowly and the dough was kneaded as hard as the ear lobe. The dough was cut into three equal pieces and kept dry by covering them in a plastic wrap. 1g of powdered tea was added to one of the cut pieces, 7g of strawberry jam mixed with another piece and nothing was added to the other. These 3 dough was spread into a bar 3cm in diameter, each of them was cut into 12 equal pieces and rounded in dumpling. The rounded dough was put and boiled in boiling water in a pan. Hot jam dumpling was pierced with a skewer, followed by powdered tea dumpling and white dumpling.

Compared with dumplings that were produced using the equal amount of sugar as 1-kestose, the product was less sweet but of the same palate feeling. With the total amount of 1-kestose and sugar at 30g, dumplings were successfully produced even when the mixing ratio of 1-kestose and sugar was changed.

### [Example of food production 3: 4 1-kestose-containing puddings, content; 2.5g/piece]

By adding 3g of pumpkin powder (steamed, dried and powdered pumpkin) mixed with 200cc of water to 60g of powdered milk for allergic diseases (Morinaga New MA1 or Meiji Nobiyaka), prepared milk was produced beforehand.

In addition, 18g of agar powder, 10g of 1-kestose and 10g of sugar were mixed, and 200cc of water was added thereto and it was kept unattended for 10 minutes. The mixture was heated by adding the above prepared milt thereto, sufficiently mixed, and the heating was stopped and then 1 drop of vanilla extract was added thereto. These ingredients were introduced to the mold, cooled and hardened.

On the other hand, 18g of granulated sugar was put in a pan and the pan was shaken while heating. When the sugar scorched, the pan was removed from the flame and 30cc of hot water was added thereto, spread to make syrup for pudding. The syrup was put to cooled and hardened puddings.

Compared with puddings that were produced using the equal amount of sugar as 1-kestose, the product was less sweet but of the same palate feeling. With the total amount of 1-kestose and sugar at 20g, puddings were successfully produced even when the mixing ratio of 1-kestose and sugar was changed.

### [Example of food production 4: 500g 1-kestose-containing ice cream, content; 2g/50g]

9g of agar powder and 20g of 1-kestose were mixed and 100cc of water was added thereto and mixed, kept unattended for 10 minutes and heated and melted. By adding 110g of powdered milk for allergic diseases (Morinaga New MA1 or Meiji Nobiyaka) mixed with 200cc of water and 100g of yellow peach puree (canned yellow peach and syrup mixed in a food processor) thereto, heating and sufficiently mixing, one drop of vanilla extract was added thereto. After putting these ingredients on a Tupperware and cooling it, they were put into the refrigerator to cool and harden ice cream. Hardened ice cream was put in a food processor to cool and harden again. About 50g of ice cream was scooped with a disher for one serving.

Compared with ice cream that was produced using the equal amount of sugar as 1-kestose, the product was less sweet but of the same palate feeling. With the total amount of 1-kestose and sugar at 20g, ice cream was successfully produced even when the mixing ratio of 1-kestose and sugar was changed.

### [Example of food production 5: 350g 1-kestose-containing cake, content; 3g/35g]

By grating 80g of peeled Dioscorea batatas soaked in vinegar and mixing 30g of sugar therewith twice (60g in total), 30g of 1-kestose was added thereto and sufficiently mixed therewith. A mixture of 70g of grated carrot, 20cc of water and 1g of sodium bicarbonate was added thereto slowly and mixed up. Next, 90g of quality rice flour was sifted and added thereto, and after sufficiently mixing the mixture, it was put and kept unattended in a mold for 20 minutes. 2 half-splittable chopsticks were put on a steamer, the above mold was placed thereon and the mixture was steamed for about 20 minutes. After the cake was cooled, it was removed from the mold and cut into pieces with about 35g of cake as one serving.

Compared with cake that was produced using the equal amount of sugar as 1-kestose, the product was less sweet but of the same palate feeling. With the total amount of 1-kestose and sugar at 90g, cake was successfully produced even when the mixing ratio of 1-kestose and sugar was changed. However, the mixing ratio used in this Example was appropriate in view of the volume of cake required.

From the results of the above Example 8, it was confirmed that various 1-kestose-containing foods can be produced. Also, if sugar is replaced with 1-kestose, the product foods provide the same level of sweetness and palate feeling and can be easily eaten by infants as well.

Example 8 shows a method for producing 1-kestose-containing foods that uses no usual eggs, milk, soybeans and wheat leading to food allergen, but Sakusaku flour and tapioca flour may be replaced with wheat flour for users with no food allergy and allergy-prone materials may be replaced with usual ones.

### "Example 9: Production of 1-kestose-containing drugs"

Next, methods for producing drugs mainly for atopic dermatitis using 1-kestose will be described.

### [Example of drug production 1: 1,000g 1-kestose-containing powder drug, content; 800mg/1g]

In Example of drug production 1, after sufficiently mixing 800g of 1-kestose and 200g of lactose, 300ml of 90% ethanol was added thereto and the mixture was put in a wet condition. After granulating wet powder, it was aerated and kept dry at 60°C for 16 hours. After drying it, powder drug of appropriate granularity was obtained by making particle size thereof.

### [Example of drug production 2: 5,000 1-kestose-containing tablets, content; 60mg/tablet]

The tablets in Example of drug production 2 can be produced by making 1-kestose-containing powder mixture, granulating and slagging it, adding a disintegrator or lubricant thereto and tableting. Specifically, after sufficiently mixing 300g of 1-kestose, 380g of powdery reduced malt sugar syrup, 180g of rice starch, 100g of dextrin, 300ml of 90% ethanol was added thereto and kept in a wet condition. After wet powder was extruded and granulated, it was aerated and kept dry at 60°C for 16 hours. After drying it, granules were granulated with an 850µm-sieve and 50g of sucrose fatty acid ester was added to 470g of granules and mixed. Then, tablets were made with a rotary tableting machine (6B-2, KIKUSUI SEISAKUSHO LTD.) to obtain 200mg of tablets 8mm in diameter.

From the results of the above Example 9, 1-kestose can be produced in the form of food and drug and it was confirmed that it can be used as pharmaceuticals.

The allergy-suppressive agent containing 1-kestose as a main ingredient as shown in Example 9 is not limited to the above conditions and it may be altered accordingly. For example, it may be taken not only once a day, but also separately twice to 4 times a day. The method for administering the allergy-suppressive agent is not specifically limited and it may be oral or rectal according to the type and condition of a patient. Also, the dosage form is not specifically limited and it may be selected according to the method for administering the allergy-suppressive agent. For example, the dosage form for oral administration can be powder drug, tablet, sugar-coated tablet, capsule, granule, dry syrup, solution, syrup, lozenge, health drink, and other solid or liquid substance.

The above-mentioned allergy-suppressive composition, allergy-suppressive food and allergy-suppressive agent of each embodiment have an effect of inhibiting the production of IgE (IgG1) antibody as an causative agent of allergy, an effect of increasing the production of IgA antibody that primarily maintains intestinal immune tolerance, an effect of activating the growth of an intestinal *Bifidobacterium* and an effect of improving the intestinal environment in the prevention and treatment of allergic diseases.

Particularly, there was a significant effect of ameliorating atopic dermatitis in infants and young children. Also, 1-kestose is a highly safe substance of low hygroscopicity and high solubility, resulting in a suitable drug for taking and drinking on a daily basis for infants and young children and high preventive effect by long-term use. By ameliorating atopic dermatitis in infancy, it is expected that allergy march in patients with atopic diathesis can be effectively prevented or inhibited.

The composition having the above effects preferably proposes a high purity of 1-kestose therein. The 1-kestose contained is required to have a higher purity larger than nystose, and the purity is preferably 90% or more, and more preferably 95 % or more.

For example, the oligosaccharide, mainly composed of 1-kestose used in this embodiment, may contain other compositions like nystose and sucrose, but it is not limited to these substances. The use of crystallized 1-kestose can be obviously achieved and 1-kestose can be mixed with any type of material with any ratio, particularly with other useful materials such as useful viable cell material and mushroom-derived polysaccharide. In addition, the oligosaccharide can be used with *Lactobacilluse* fermented food like yogurt. It can be taken in the form of powder, its melt, tablet, capsule or others. For example, using 50 parts by weight of 1-kestose, 30 parts by weight of dextrin and 20 parts by weight of vegetable oil, 1-kestose may be contained in tablet candies for anti-atopic dermatitis according to a conventional production method.

## Claims

1. A composition comprising oligosaccharide with 1-kestose as main ingredient for use in the prevention or treatment of allergy, wherein the amount of 1-kestoseis larger than that of nystose.

2. The composition for use according to claim 1, wherein the composition is used for increasing the production of IgA antibody.

3. The composition for use according to any of claims 1 or 2 for inhibiting the production of IgE antibody.

4. The composition for use according to any of claims 1 to 3 for activating the growth of an enteral *Bifidobacterium* in infants and young children.

5. A composition comprising oligosaccharide with 1-kestose as main ingredient for use in ameliorating atopic dermatitis in infants and young children, wherein the amount of 1-kestose is larger than that of nystose.

6. The composition for use as set forth in any one of the Claims 1 to 5,
wherein said 1-kestose is a crystallized kestose with a purity of 95 % or more.

7. The composition for use as set forth in any one of Claims 1 to 6,
which is formulated at a daily effective dose of 0.015 (g/kg body weight) or more.

8. The composition for use as set forth in any one of Claims 1 to 7,
wherein the composition is used as food.

## Patentansprüche

1. Zusammensetzung, die Oligosaccharid mit 1-Kestose als Hauptbestandteil umfasst, zur Verwendung bei der Prävention oder Behandlung von Allergie, wobei die Menge an 1-Kestose größer als die von Nystose ist.

2. Zusammensetzung zur Verwendung gemäß Anspruch 1, wobei die Zusammensetzung zur Steigerung der Produktion von IgA-Antikörper verwendet wird.

3. Zusammensetzung zur Verwendung gemäß irgendeinem der Ansprüche 1 oder 2 zur Inhibierung der Produktion von IgE-Antikörper.

4. Zusammensetzung zur Verwendung gemäß irgendeinem der Ansprüche 1 bis 3 zur Aktivierung des Wachstums eines enteralen *Bifidobacteriums* bei Kleinkindern und jungen Kindern.

5. Zusammensetzung, die Oligosaccharid mit 1-Kestose als Hauptbestandteil umfasst, zur Verwendung bei der Verbesserung atopischer Dermatitis bei Kleinkindern und jungen Kindern, wobei die Menge an 1-Kestose größer als die von Nystose ist.

6. Zusammensetzung zur Verwendung wie in irgendeinem der Ansprüche 1 bis 5 dargelegt, wobei die 1-Kestose eine kristallisierte Kestose mit einer Reinheit von 95% oder mehr ist.

7. Zusammensetzung zur Verwendung wie in irgendeinem der Ansprüche 1 bis 6 dargelegt, welche mit einer täglichen wirksamen Dosis von 0,015 (g/kg Körpergewicht) oder mehr formuliert ist.

8. Zusammensetzung zur Verwendung wie in irgendeinem der Ansprüche 1 bis 7 dargelegt, wobei die Zusammensetzung als Nahrungsmittel verwendet wird.

## Revendications

1. Composition à teneur en oligosaccharide contenant du 1-kestose comme ingrédient principal, pour une utilisation dans la prévention ou le traitement d'une allergie, dans laquelle la quantité de 1-kestose est supérieure à celle du nystose.

2. Composition pour une utilisation selon la revendication 1, dans laquelle la composition est utilisée pour augmenter la production d'anticorps de type IgA.

3. Composition pour une utilisation selon l'une quelconque des revendications 1 ou 2, pour inhiber la production d'anticorps de type IgE.

4. Composition pour une utilisation selon l'une quelconque des revendications 1 à 3, pour activer la croissance d'une bactérie entérale du genre *Bifidobacterium* chez les nourrissons et les jeunes enfants.

5. Composition à teneur en oligosaccharide comprenant du 1-kestose comme ingrédient principal, pour une utilisation dans le but d'améliorer une dermatite atopique chez les nourrissons et les jeunes enfants, dans laquelle la quantité de 1-kestose est supérieure à celle du nystose.

6. Composition pour une utilisation selon l'une quelconque des revendications 1 à 5, dans laquelle ledit 1-kestose est un kestose cristallisé présentant une pureté de 95 % ou plus.

7. Composition pour une utilisation selon l'une quelconque des revendications 1 à 6, qui est formulée à une dose quotidienne efficace de 0,015 (g/kg de poids corporel) ou plus.

8. Composition pour une utilisation selon l'une quelconque des revendications 1 à 7, dans laquelle la composition est utilisée comme produit alimentaire.
